# FASCICULE DE BREVET EUROPEEN

(11) **EP 2 887 910 B1**
(45) Date de publication et mention de la délivrance du brevet: **24.08.2016**
(21) Numéro de dépôt: 13758911.5
(22) Date de dépôt: 25.07.2013
(51) Int. Cl.: A61F 2/38

(54) **EMBASE TIBIALE PROTHÉTIQUE ET INSERT TIBIAL PROTHÉTIQUE DESTINE A ÊTRE IMMOBILISÉ SUR UNE TELLE EMBASE TIBIALE PROTHÉTIQUE**
TIBIAPROTHESENBASIS UND TIBIAPROTHESENEINSATZ ZUR IMMOBILISIERUNG AUF EINER DERARTIGEN TIBIAPROTHESENBASIS
PROSTHETIC TIBIAL BASE, AND PROSTHETIC TIBIAL INSERT WHICH IS TO BE IMMOBILIZED ON SUCH A PROSTHETIC TIBIAL BASE

(30) Priorité: 24.08.2012 FR 1257982
(43) Date de publication de la demande: 01.07.2015
(73) Titulaire: Anatomic, 13011 Marseille (FR)
(72) Inventeur: ROISIN, Alain, F-92430 Marnes La Coquette (FR); VAN HILLE, William, F-63960 Veyre Monton (FR); YVROUD, Michel, F-57420 Coin Les Cuvry (FR); PERONNE, Etienne, F-63400 Chamalieres (FR); PIRIOU, Philippe, F-95270 Belloy En France (FR); GUINGAND, Olivier, F-75016 Paris (FR); BORRIONE, Frederic, F-13011 Marseille (FR); OGET, Vincent, F-51100 Reims (FR); PITON, Jean-Pierre, F-57640 Sanry Les Vigy (FR); LHOTELLIER, Luc, 94120 Fontenay sous Bois (FR); GUYEN, Olivier, F-69003 Lyon (FR); BONNOMET, François, F-67205 Oberhausbergen (FR); POTEL, Jean-François, F-31500 Toulouse (FR); BOUSSATON, Michel, F-31120 Roques Sur Garonne (FR)
(74) Mandataire: Chevalier, Renaud Philippe
(86) Numéro de dépôt international: PCT/FR2013/051792
(87) Numéro de publication internationale: WO 2014/029928

(56) Documents cités:
- EP-A1- 2 042 134
- EP-A2- 2 042 131
- WO-A1-01/41680
- FR-A1- 2 719 213

## Description

La présente invention concerne une embase tibiale prothétique destinée à immobiliser un insert tibial prothétique. De plus, la présente invention concerne un insert tibial prothétique destiné à être immobilisé sur une telle embase tibiale prothétique.

La présente invention trouve notamment application dans le domaine des prothèses de genou, en particulier pour le remplacement de la partie proximale du tibia. L'état de la technique le plus proche est donné par le document EP 2042134 A1, qui définit les préambules des revendications indépendantes 1 et 10.

Dans l'art antérieur, il existe une embase tibiale comprenant, d'une part, des moyens de fixation sur une partie réséquée du tibia d'un patient et, d'autre part, une platine dont les bords latéral et médial présentent des ergots pour y assembler un insert tibial. L'embase tibiale est généralement en alliage métallique et l'insert tibial est généralement en matériau polymère de synthèse, tel qu'un polyéthylène.

Cependant, on a constaté que la fixation d'un insert tibial sur une telle embase tibiale produisait un certain jeu, en particulier suivant une direction antéropostérieure, ce qui induit des frottements de l'insert tibial sur l'embase tibiale en service. L'usure causée par ces frottements risque de produire de petits débris d'insert tibial, lesquels risquent de causer une ostéolyse.

La présente invention vise notamment à résoudre, en tout ou partie, les problèmes mentionnés ci-avant.

À cet effet, l'invention a pour objet une embase tibiale prothétique comme définie dans la revendication 1.

Ainsi, une telle embase tibiale prothétique permet de fixer un insert tibial prothétique sans jeu ou avec un jeu insignifiant, en particulier suivant la direction antéropostérieure, ce qui évite tout jeu dans la prothèse et donc l'usure prématurée de l'insert tibial prothétique.

Selon un mode de réalisation de l'invention, la distance séparant le méplat postérieur du méplat antérieur, mesurée suivant une direction antéropostérieure, est déterminée de façon à réaliser un emmanchement de l'insert tibial prothétique.

Ainsi, un tel emmanchement permet de fixer fermement l'insert tibial prothétique sur l'embase tibiale prothétique.

Selon un mode de réalisation de l'invention, l'état de surface du plateau présente un écart moyen arithmétique inférieur à 0,1 µm, de préférence inférieur à 0,05 µm.

En d'autres termes, le plateau présente un poli miroir.

Ainsi, un tel plateau lisse évite l'usure de l'insert tibial prothétique même lorsque sa fixation présente un jeu insignifiant.

Selon un mode de réalisation de l'invention, le plateau présente une surface de plateau plane, la surface de plateau étant de préférence perpendiculaire à l'ergot postérieur, à l'ergot antérieur, aux pattes postérieures et aux pattes antérieures.

Ainsi, une telle surface de plateau permet de répartir, uniformément et sur une grande superficie, les efforts transmis entre l'insert tibial prothétique et l'embase tibiale prothétique.

Selon un mode de réalisation de l'invention, le méplat postérieur et le méplat antérieur sont plans et parallèles entre eux, le méplat postérieur et le méplat antérieur étant de préférence perpendiculaires à une direction antéropostérieure.

Ainsi, de tels méplats assurent un maintien de l'insert tibial prothétique sur l'embase tibiale prothétique suivant la direction antéropostérieure.

Selon un mode de réalisation de l'invention, le méplat postérieur et le méplat antérieur sont agencés de sorte que l'insert tibial prothétique peut être inséré en ajustement serré entre le méplat postérieur et le méplat antérieur.

Ainsi, un tel ajustement serré assure un maintien optimal de l'insert tibial prothétique sur l'embase tibiale prothétique.

Selon un mode de réalisation de l'invention, chaque extension de maintien a une section transversale en forme de queue d'aronde.

Ainsi, de telles extensions de maintien permettent de fixer rapidement l'insert tibial prothétique sur l'embase tibiale prothétique.

Selon un mode de réalisation de l'invention, la platine est composée d'un matériau sélectionné dans le groupe constitué d'un alliage de chrome-cobalt, d'un acier inoxydable de type 316L ou de type M30NW, et d'un alliage de titane TA6V4Eli.

Ainsi, un tel matériau permet de réaliser une embase tibiale prothétique présentant un faible coefficient de frottement.

Selon un mode de réalisation de l'invention, un film de protection recouvre au moins le plateau, le film de protection étant amovible, de façon à être retiré lors de l'intervention chirurgicale avant impaction définitive de l'insert tibial prothétique sur l'embase tibiale prothétique.

Ainsi, un tel film de protection permet de protéger l'embase avant l'intervention chirurgicale.

Par ailleurs, la présente invention a pour objet un insert tibial prothétique, destiné à être immobilisé sur une embase tibiale prothétique, comme défini dans la revendicchon 10.

Ainsi, un tel insert tibial peut être fixé sur une embase tibiale prothétique conforme à l'invention sans jeu ou avec un jeu insignifiant, en particulier suivant la direction antéropostérieure, ce qui évite tout jeu dans la prothèse et donc l'usure prématurée de l'insert tibial prothétique.

Selon un mode de réalisation de l'invention, au moins la portion inférieure est composée d'un matériau sélectionné dans le groupe constitué d'un polyéthylène à ultrahaut poids moléculaire (UHMW-PE), de polyuréthane, et dans lequel la portion inférieure a une épaisseur supérieure à 1,5 mm.

Ainsi, un tel matériau permet de réaliser un insert tibial prothétique présentant un faible coefficient de frottement.

Les modes de réalisation et les variantes mentionnés ci-avant peuvent être pris isolément ou selon toute combinaison techniquement admissible.

La présente invention sera bien comprise et ses avantages ressortiront aussi à la lumière de la description qui va suivre, donnée uniquement à titre d'exemple non limitatif et faite en référence aux dessins annexés, dans lesquels :
- la figure 1 est une vue en perspective antérieure d'une embase tibiale prothétique conforme à l'invention ;
- la figure 2 est une vue en perspective postérieure de l'embase tibiale prothétique de la figure 1 ;
- la figure 3 est une vue en perspective postérieure d'un insert tibial prothétique conforme à l'invention et destiné à être immobilisé sur l'embase tibiale prothétique des figures 1 et 2 ;
- la figure 4 est une vue en perspective antérieure de l'insert tibial prothétique de la figure 3 ;

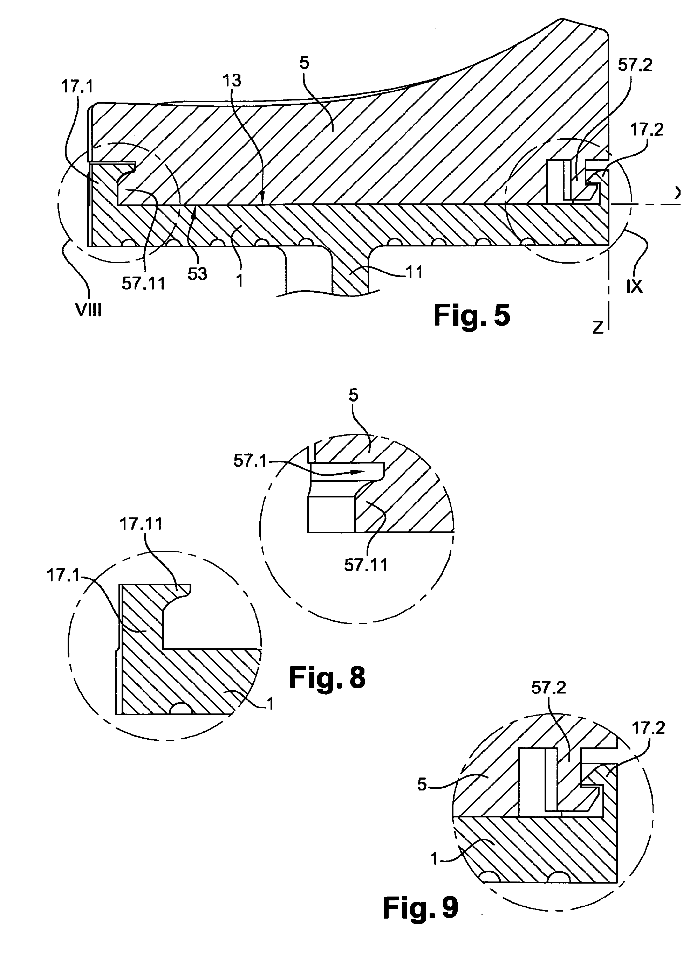

- la figure 5 est une vue en coupe suivant le plan V à la figure 1 et illustrant l'insert tibial prothétique des figures 3 et 4 immobilisé sur l'embase tibiale prothétique des figures 1 et 2 ;

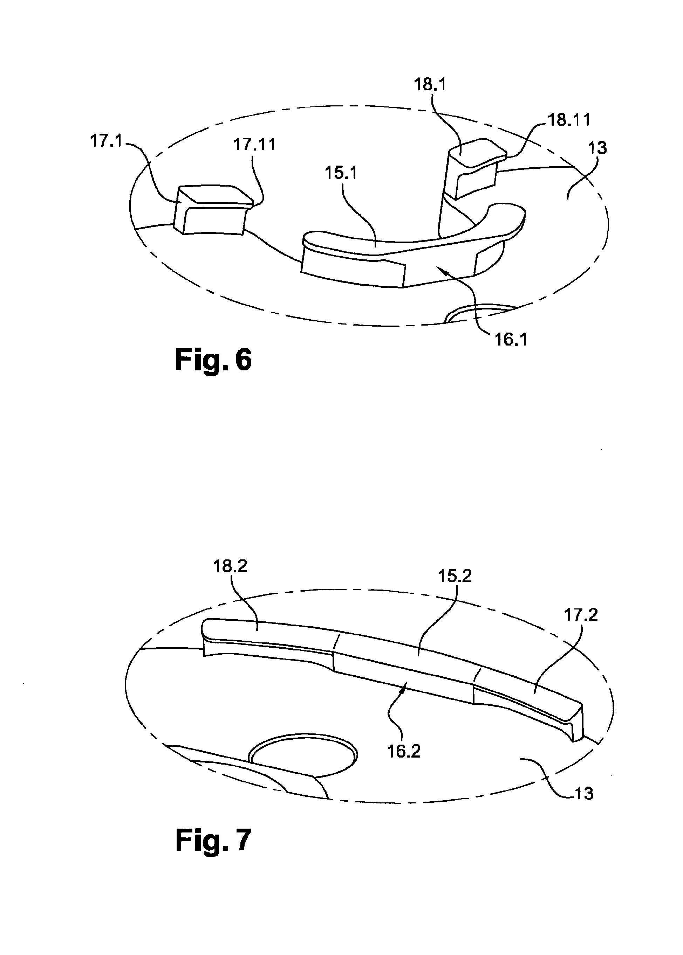

- la figure 6 est une vue à plus grande échelle du détail VI à la figure 1 ;
- la figure 7 est une vue à plus grande échelle du détail VII à la figure 2 ;
- la figure 8 est une vue éclatée à plus grande échelle du détail VIII à la figure 5 ; et
- la figure 9 est une vue à plus grande échelle du détail IX à la figure 5.

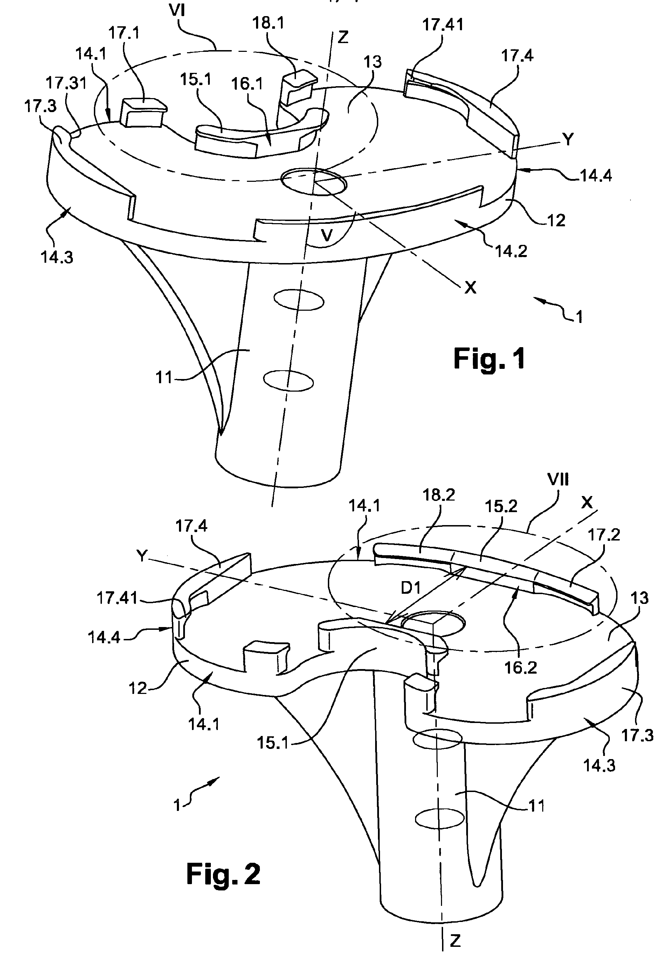

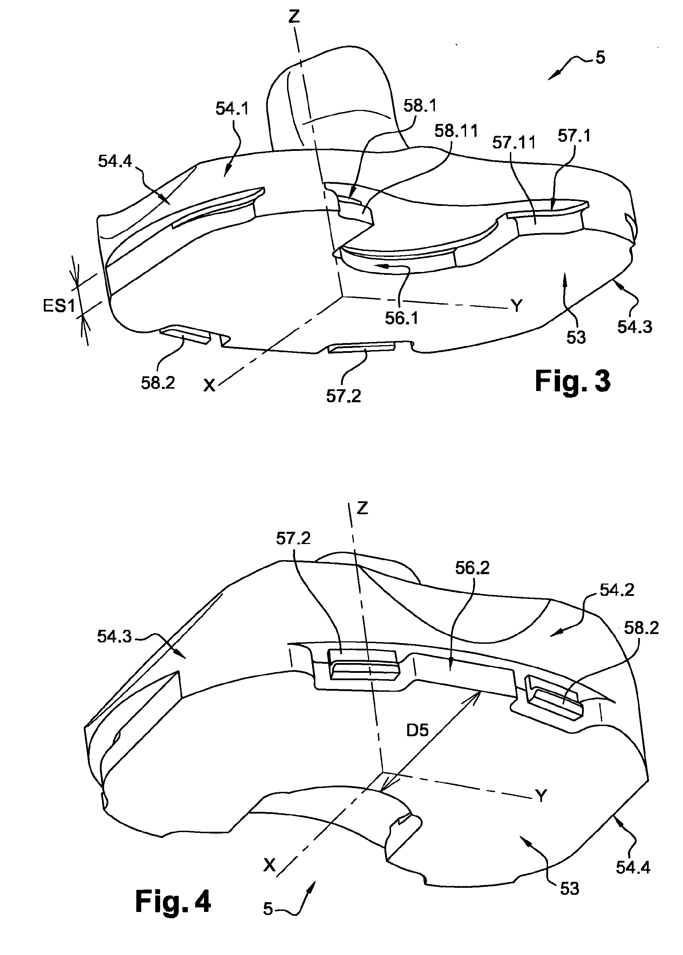

Les figures 1 et 2 illustrent une embase tibiale prothétique 1, qui est destinée à immobiliser un insert tibial prothétique 5, illustré aux figures 3 et 4. L'insert tibial prothétique 5 est conformé pour s'articuler avec des condyles fémoraux non représentés.

L'embase tibiale prothétique 1 comprend une platine 12 et des moyens de fixation osseuse 11 pour fixer l'embase tibiale prothétique 1 sur une partie réséquée du tibia d'un patient.

La platine 12 comprend un plateau 13 qui est conformé pour recevoir une face inférieure complémentaire 53 de l'insert tibial prothétique 5. Le plateau 13 est délimité par un bord postérieur 14.1, par un bord antérieur 14.2 opposé au bord postérieur 14.1, par un bord médial 14.3 et par un bord latéral 14.4 opposé au bord médial 14.3.

La platine 12 comprend aussi un ergot postérieur 15.1 qui fait saillie au-dessus du plateau 13, d'environ 2 mm. L'ergot postérieur 15.1 est disposé, suivant une direction médio-latérale Y, sur une région médiane du bord postérieur 14.1. L'ergot postérieur 15.1 présente un méplat postérieur 16.1 orienté vers le bord antérieur 14.2 et parallèlement au plan frontal. Le plan frontal sépare le corps en une partie antérieure et une partie postérieure. Sur les figures 1 et 2, le plan YZ est parallèle au plan frontal. La direction Z est une direction sagittale.

La platine 12 comprend en outre un ergot antérieur 15.2 qui fait saillie au-dessus du plateau 13, d'environ 2mm. L'ergot antérieur 15.2 est disposé, suivant une direction médio-latérale Y, sur une région médiane du bord antérieur 14.2. L'ergot antérieur 15.2 présentant un méplat antérieur 16.2 orienté vers le bord postérieur 14.1 et parallèlement au plan frontal, donc au plan YZ.

De plus, la platine 12 comprend deux pattes postérieures 17.1 et18.1 qui font saillie au-dessus du plateau 13, d'environ 2 mm. Les deux pattes postérieures 17.1, 18.1 sont disposées sur le bord postérieur 14.1 et respectivement de chaque côté de l'ergot postérieur 15.1.

De même, la platine 12 comprend deux pattes antérieures 17.2 et 18.2 qui font saillie au-dessus du plateau 13, d'environ 2 mm. Les deux pattes antérieures 17.2, 18.2 sont disposées sur le bord antérieur 14.2 et respectivement de chaque côté de l'ergot antérieur 15.2.

Le méplat postérieur 16.1 et le méplat antérieur 16.2 sont agencés de sorte que l'insert tibial prothétique 5 peut s'appuyer simultanément contre le méplat postérieur 16.1 et contre le méplat antérieur 16.2.

Chaque patte postérieure 17.1 ou 18.1 comporte une extension de maintien respective 17.11 et 18.11 pour maintenir l'insert tibial prothétique 5 sur l'embase tibiale prothétique 1. Chaque extension de maintien 17.11 ou 18.11 est conformée pour permettre l'insertion, sous l'extension de maintien 17.11 ou 18.11 et suivant une direction antéropostérieure, d'une languette de maintien complémentaire 57.11 ou 58.11 qui appartient à l'insert tibial prothétique 5.

Dans l'exemple des figures 1 à 4, chaque extension de maintien 17.11 ou 18.11 a une section transversale en forme de queue d'aronde, ici en « J » renversé.

Chaque patte antérieure 17.2 ou 18.2 comporte des moyens d'encliquetage élastique de l'insert tibial prothétique 5 sur l'embase tibiale prothétique 1.

Les figures 3 et 4 illustrent l'insert tibial prothétique 5, qui est destiné à être immobilisé sur l'embase tibiale prothétique 1. L'insert tibial prothétique 5 présente des surfaces conformées pour s'articuler avec des condyles fémoraux respectifs non représentés.

L'insert tibial prothétique 5 a une portion inférieure 51 qui comprend une face inférieure 53 conformée pour être apposée sur un plateau 13 complémentaire de l'embase tibiale prothétique 1. La face inférieure 53 est délimitée par un côté postérieur 54.1, par un côté antérieur 54.2 opposé au côté postérieur 54.1, par un côté médial 54.3 et par un côté latéral 54.4 opposé au côté médial 54.3.

La portion inférieure 51 de l'insert tibial prothétique 5 comprend aussi :
- un plat postérieur 56.1 qui est ménagé sur le côté postérieur 54.1 ; le plat postérieur 56.1 est disposé, suivant une direction médio-latérale Y, sur une région médiane du côté postérieur 54.1 ; le plat postérieur 56.1 est orienté vers le côté antérieur 54.2 et parallèlement au plan frontal, donc au plan YZ ;
- un plat antérieur 56.2 ménagé sur le côté antérieur 54.2 ; le plat antérieur 54.2 est disposé, suivant une direction médio-latérale Y, sur une région médiane du côté antérieur 54.2 ; le plat antérieur 56.2 est orienté vers le côté postérieur 54.1 et parallèlement au plan frontal, donc au plan YZ ; et
- deux logements postérieurs 57.1 et 58.1 ménagés sur le côté postérieur 54.1, les deux logements postérieurs 57.1 et 58.1 de part et d'autre du plat postérieur 56.1.

Lorsque l'insert tibial prothétique 5 est immobilisé sur l'embase tibiale prothétique 1, les plats postérieur 56.1 et antérieur 56.2 coopèrent respectivement avec les méplats postérieur 16.1 et antérieur 16.2. Pour obtenir cette coopération, pour un insert tibial de taille 4 dans une gamme d'inserts tibiaux, la cote entre les surfaces 56.1 et 56.2 est de 29,57mm -0,1/- 0,2 mm et la cote entre les méplats 16.1 et 16.2 est de 30,17 mm 0/-0.6 mm. Le jeu minimal est donc de 0 mm et le jeu maximal est de 0,8 mm.

De plus, la portion inférieure 51 de l'insert tibial prothétique 5 comprend deux organes antérieurs 57.2 et 58.2 ménagés sur le côté antérieur 54.2. Les deux organes antérieurs 57.2 et 58.2 sont disposés sur le côté antérieur 54.2 et respectivement de part et d'autre du plat antérieur 56.2.

Le plat postérieur 56.1 et le plat antérieur 56.2 sont agencés de sorte que l'embase tibiale prothétique 1 peut s'appuyer simultanément contre le plat postérieur 56.1 et contre le plat antérieur 56.2.

Chaque logement postérieur 57.1 ou 58.1 comporte une languette de maintien 57.11 ou 58.11 pour maintenir l'insert tibial prothétique 5 sur l'embase tibiale prothétique 1. Chaque languette de maintien 57.11 ou 58.11 est conformée pour être insérée, suivant une direction antéropostérieure X, sous une extension de maintien complémentaire 17.11 ou 18.11 qui appartient à l'embase tibiale prothétique 1.

Chaque organe antérieur 57.2 ou 58.2 comporte des moyens d'encliquetage élastique de l'embase tibiale prothétique 1 sur l'insert tibial prothétique 5.

Par ailleurs, la distance D1 entre le méplat postérieur 16.1 et le méplat antérieur 16.2, mesurée suivant une direction antéropostérieure X, est déterminée de façon à réaliser un emmanchement de l'insert tibial prothétique 5. De manière complémentaire, la distance D5 entre le plat postérieur 56.1, mesurée suivant une direction antéropostérieure X, est déterminée de façon à réaliser un emmanchement de l'insert tibial prothétique 5. Dans l'exemple des figures 1 à 4, la distance D1 mesure 30,17 mm 0/-0,6 mm et la distance D5 mesure 29,57 mm -0,1/-0,2 mm.

L'embase tibiale prothétique 1 comprend en outre :
- une patte médiale 17.3 qui fait saillie au-dessus du plateau 13, d'envrion 2 mm ; la patte médiale 17.3 est disposée sur le bord médial 14.3 ; la patte médiale 17.3 comporte une aile de maintien 17.31 pour maintenir l'insert tibial prothétique 5 sur l'embase tibiale prothétique 1 ; l'aile de maintien 17.31 est conformée pour permettre l'insertion, sous l'aile de maintien et suivant une direction médio-latérale Y d'un tenon de maintien complémentaire qui appartient à l'insert tibial prothétique 5 ; et
- une patte latérale 17.4 qui fait saillie au-dessus du plateau 13 ; la patte latérale 17.4 est disposée sur le bord latéral 14.4 ; la patte latérale 17.4 comporte une aile de maintien 17.41 pour maintenir l'insert tibial prothétique 5 sur l'embase tibiale prothétique 1 ; l'aile de maintien 17.41 est conformée pour permettre l'insertion, sous l'aile de maintien 17.41 et suivant une direction médio-latérale Y d'un tenon de maintien complémentaire qui appartient à l'insert tibial prothétique 5.

Par ailleurs, l'état de surface du plateau 13 présente un écart moyen arithmétique (Ra) qui est d'environ 0,05 µm. Le plateau 13 a donc un état de surface en poli miroir.

De plus, le plateau 13 présente une surface de plateau qui est plane et qui est ici perpendiculaire à l'ergot postérieur 15.1, à l'ergot antérieur 15.2, aux pattes postérieures 17.1 et 18.1 et aux pattes antérieures 17.2 et 18.2.

Le méplat postérieur 16.1 et le méplat antérieur 16.2 sont plans et parallèles entre eux et ils sont ici perpendiculaires à la direction antéropostérieure X. Le méplat postérieur 16.1 et le méplat antérieur 16.2 sont agencés de sorte que l'insert tibial prothétique 5 peut être inséré en ajustement serré entre le méplat postérieur 16.1 et le méplat antérieur 16.2.

La platine 12 de l'embase tibiale prothétique 1 est ici composée d'un alliage de chrome-cobalt. La portion inférieure 51 de l'insert tibial prothétique 5 est ici composée d'un polyéthylène à ultrahaut poids moléculaire (UHMW-PE). La portion inférieure 51 a ici une épaisseur E51 de 3,5mm +/- 0,05mm.

On peut prévoir un film de protection recouvrant le plateau 13. Ce film de protection est amovible, de façon à être retiré lors de l'intervention chirurgicale avant impaction définitive de l'insert tibial prothétique 5 sur l'embase tibiale prothétique 1.

## Revendications

1. Embase tibiale prothétique (1), destinée à immobiliser un insert tibial prothétique (5) conformé pour s'articuler avec des condyles fémoraux, l'embase tibiale prothétique (1) comprenant i) des moyens de fixation osseuse (11) pour fixer l'embase tibiale prothétique (1) sur une partie réséquée du tibia d'un patient, et ii) une platine (12) comprenant au moins :
- un plateau (13) conformé pour recevoir une face inférieure complémentaire (53) de l'insert tibial prothétique (5), le plateau (13) étant délimité par un bord postérieur (14.1), par un bord antérieur (14.2) opposé au bord postérieur (14.1), par un bord médial (14.3) et par un bord latéral (14.4) opposé au bord médial (14.3) ;
- un ergot postérieur (15.1) faisant saillie au-dessus du plateau (13), l'ergot postérieur (15.1) étant disposé, suivant une direction médio-latérale (Y), sur une région médiane du bord postérieur (14.1), l'ergot postérieur (15.1) présentant un méplat postérieur (16.1) orienté vers le bord antérieur (14.2), de préférence parallèlement au plan frontal (YZ) ;
- un ergot antérieur (15.2) faisant saillie au-dessus du plateau (13), l'ergot antérieur (15.2) étant disposé, suivant une direction médio-latérale (Y), sur une région médiane du bord antérieur (14.2), l'ergot antérieur (15.2) présentant un méplat antérieur (16.2) orienté vers le bord postérieur (14.1), de préférence parallèlement au plan frontal (YZ) ;
- deux pattes postérieures (17.1, 18.1) faisant saillie au-dessus du plateau (13), les deux pattes postérieures (17.1, 18.1) étant disposées sur le bord postérieur (14.1) et respectivement de chaque côté de l'ergot postérieur (15.1) ;
- deux pattes antérieures (17.2, 18.2) faisant saillie au-dessus du plateau (13), les deux pattes antérieures (17.2, 18.2) étant disposées sur le bord antérieur (14.2) et respectivement de chaque côté de l'ergot antérieur (15.2) ;
le méplat postérieur (16.1) et le méplat antérieur (16.2) sont agencés de sorte que l'insert tibial prothétique (5) peut s'appuyer simultanément contre le méplat postérieur (16.1) et contre le méplat antérieur (16.2) ;
chaque patte postérieure (17.1, 18.1) comporte une extension de maintien respective (17.11, 18.11) pour maintenir l'insert tibial prothétique (5) sur l'embase tibiale prothétique (1), chaque extension de maintien (17.11, 18.11) étant conformée pour permettre l'insertion, sous l'extension de maintien (17.11, 18.11) et suivant une direction antéropostérieure, d'une languette de maintien complémentaire (57.11, 58.11) appartenant à l'insert tibial prothétique (5) ;
chaque patte antérieure (17.2, 18.2) comporte des moyens d'encliquetage élastique de l'insert tibial prothétique (5) sur l'embase tibiale prothétique (1) ;
l'embase tibiale prothétique (1) étant **caractérisée en ce qu'**elle comprend en outre :
- une patte médiale (17.3) faisant saillie au-dessus du plateau (13), la patte médiale (17.3) étant disposée sur le bord médial (14.3), la patte médiale (17.3) comportant une aile de maintien (17.31) pour maintenir l'insert tibial prothétique (5) sur l'embase tibiale prothétique (1), l'aile de maintien (17.31) étant conformée pour permettre l'insertion, sous l'aile de maintien et suivant une direction médio-latérale (Y) d'un tenon de maintien complémentaire appartenant à l'insert tibial prothétique (5) ; et
- une patte latérale (17.4) faisant saillie au-dessus du plateau (13), la patte latérale (17.4) étant disposée sur le bord latéral (14.4), la patte latérale (17.4) comportant une aile de maintien (17.41) pour maintenir l'insert tibial prothétique (5) sur l'embase tibiale prothétique (1), l'aile de maintien (17.41) étant conformée pour permettre l'insertion, sous l'aile de maintien (17.41) et suivant une direction médio-latérale (Y) d'un tenon de maintien complémentaire appartenant à l'insert tibial prothétique (5).

2. Embase tibiale prothétique (1) selon la revendication 1, dans laquelle la distance (D1) séparant le méplat postérieur (16.1) du méplat antérieur (16.2), mesurée suivant une direction antéropostérieure (X), est déterminée de façon à réaliser un emmanchement de l'insert tibial prothétique (5).

3. Embase tibiale prothétique (1) selon l'une des revendications précédentes, dans laquelle l'état de surface du plateau (13) présente un écart moyen arithmétique inférieur à 0,1 µm, de préférence inférieur à 0,05 µm.

4. Embase tibiale prothétique (1) selon l'une des revendications précédentes, dans laquelle le plateau (13) présente une surface de plateau plane, la surface de plateau étant de préférence perpendiculaire à l'ergot postérieur (15.1), à l'ergot antérieur (15.2), aux pattes postérieures (17.1, 18.1) et aux pattes antérieures (17.2, 18.2).

5. Embase tibiale prothétique (1) selon l'une des revendications précédentes, dans laquelle le méplat postérieur (16.1) et le méplat antérieur (16.2) sont plans et parallèles entre eux, le méplat postérieur (16.1) et le méplat antérieur (16.2) étant de préférence perpendiculaires à une direction antéropostérieure (X).

6. Embase tibiale prothétique (1) selon la revendication 5, dans laquelle le méplat postérieur (16.1) et le méplat antérieur (16.2) sont agencés de sorte que l'insert tibial prothétique (5) peut être inséré en ajustement serré entre le méplat postérieur (16.1) et le méplat antérieur (16.2).

7. Embase tibiale prothétique (1) selon l'une des revendications précédentes, dans laquelle chaque extension de maintien (17.11, 18.11) a une section transversale en forme de queue d'aronde.

8. Embase tibiale prothétique (1) selon l'une des revendications précédentes, dans laquelle la platine (12) est composée d'un matériau sélectionné dans le groupe constitué d'un alliage de chrome-cobalt, d'un acier inoxydable de type 316L ou de type M30NW, et d'un alliage de titane TA6V4Eli.

9. Embase tibiale prothétique (1) selon l'une des revendications précédentes, dans laquelle un film de protection recouvre au moins le plateau (13), le film de protection étant amovible, de façon à être retiré lors de l'intervention chirurgicale avant impaction définitive de l'insert tibial prothétique (5) sur l'embase tibiale prothétique (1).

10. Insert tibial prothétique (5), destiné à être immobilisé sur une embase tibiale prothétique (1), l'insert tibial prothétique (5) présentant des surfaces conformées pour s'articuler avec des condyles fémoraux respectifs, l'insert tibial prothétique (5) ayant une portion inférieure (51) comprenant :
- une face inférieure (53) conformée pour être apposée sur un plateau (13) complémentaire de l'embase tibiale prothétique (1), la face inférieure (53) étant délimitée par un côté postérieur (54.1), par un côté antérieur (54.2) opposé au côté postérieur (54.1), par un côté médial (54.3) et par un côté latéral (54.4) opposé au côté médial (54.3) ;
- un plat postérieur (56.1) ménagé sur le côté postérieur (54.1), le plat postérieur (56.1) étant disposé, suivant une direction médio-latérale (Y), sur une région médiane du côté postérieur (54.1), le plat postérieur (56.1) étant orienté vers le côté antérieur (54.2), de préférence parallèlement au plan frontal (YZ) ;
- un plat antérieur (56.2) ménagé sur le côté antérieur (54.2), le plat antérieur (54.2) étant disposé, suivant une direction médio-latérale (Y), sur une région médiane du côté antérieur (54.2), le plat antérieur (56.2) étant orienté vers le côté postérieur (54.1), de préférence parallèlement au plan frontal (YZ) ;
- deux logements postérieurs (57.1, 58.1) ménagés sur le côté postérieur (54.1), les deux logements postérieurs (57.1, 58.1) de part et d'autre du plat postérieur (56.1) ;
- deux organes antérieurs (57.2, 58.2) ménagés sur le côté antérieur (54.2), les deux organes antérieurs (57.2, 58.2) étant disposés sur le côté antérieur (54.2) et respectivement de part et d'autre du plat antérieur (56.2) ;
le plat postérieur (56.1) et le plat antérieur (56.2) sont agencés de sorte que l'embase tibiale prothétique (1) peut s'appuyer simultanément contre le plat postérieur (56.1) et contre le plat antérieur (56.2) ;
chaque logement postérieur (57.1, 58.1) comporte une languette de maintien (57.11, 58.11) pour maintenir l'insert tibial prothétique (5) sur l'embase tibiale prothétique (1), chaque languette de maintien (57.11, 58.11) étant conformée pour être insérée, suivant une direction antéropostérieure (X), sous une extension de maintien complémentaire (17.11, 18.11) appartenant à l'embase tibiale prothétique (1) ;
chaque organe antérieur (57.2, 58.2) comporte des moyens d'encliquetage élastique de l'embase tibiale prothétique (1) sur l'insert tibial prothétique (5) ;
l'insert tibial prothétique (5) étant **caractérisé en ce qu'**il comprend en outre :
- un tenon de maintien médial configuré pour s'insérer, suivant une direction médio-latérale (Y), sous une aile de maintien (17.31) complémentaire appartenant à une patte médiale (17.3) d'une embase tibiale prothétique (1), et
- un tenon de maintien latéral configuré pour s'insérer, suivant une direction médio-latérale (Y), sous une aile de maintien (17.31) complémentaire appartenant à une patte latérale (17.4) d'une embase tibiale prothétique (1).

11. Insert tibial prothétique (5) selon la revendication 10, dans lequel au moins la portion inférieure (51) est composée d'un matériau sélectionné dans le groupe constitué d'un polyéthylène à ultrahaut poids moléculaire (UHMW-PE), de polyuréthane, et dans lequel la portion inférieure (51) a une épaisseur (E51) supérieure à 1,5 mm.

## Patentansprüche

1. Prothetischer Schienbeinsockel (1), der dazu bestimmt ist, einen prothetischen Schienbeineinsatz (5) stillzustellen, der ausgeformt ist, um sich an Femurkondylen anzulenken, wobei der prothetische Schienbeinsockel (1) i) Mittel zur Knochenbefestigung (11) umfasst, um den prothetischen Schienbeinsockel (1) auf einem entfernten Teil des Schienbeins eines Patienten zu befestigen, und ii) eine Platte (12), die mindestens Folgendes umfasst:
- eine Platte (13), die ausgebildet ist, um eine Innenseite (53), die zu dem prothetischen Schienbeineinsatz (5) komplementär ist, aufzunehmen, wobei die Platte (13) durch einen hinteren Rand (14.1), durch einen vorderen Rand (14.2), der dem hinteren Rand (14.1) entgegengesetzt ist, durch einen medialen Rand (14.3) und durch einen seitlichen Rand (14.4), der dem medialen Rand (14.3) entgegengesetzt ist, abgegrenzt ist;
- wobei ein hinterer Dorn (15.1) oberhalb der Platte (13) vorsteht, wobei der hintere Dorn (15.1) entlang einer medio-lateralen Richtung (Y) auf einem medianen Bereich des hinteren Rands (14.1) angeordnet ist, wobei der hintere Dorn (15.1) ein hinteres Flachteil (16.1) aufweist, das zu dem vorderen Rand (14.2) vorzugsweise parallel zu der Frontalebene (YZ) ausgerichtet ist;
- einen vorderen Dorn (15.2), der oberhalb der Platte (13) vorsteht, wobei der vordere Dorn (15.2) entlang einer medio-lateralen Richtung (Y) auf einem medianen Bereich des vorderen Rands (14.2) angeordnet ist, wobei der vordere Dorn (15.2) ein vorderes Flachteil (16.2), das zu dem hinteren Rand (14.1) vorzugsweise parallel zu der Frontalebene (YZ) ausgerichtet ist, aufweist;
- zwei hintere Pratzen (17.1, 18.1), die oberhalb der Platte (13) vorstehen, wobei die zwei hinteren Pratzen (17.1, 18.1) auf dem hinteren Rand (14.1) und jeweils auf jeder Seite des hinteren Dorns (15.1) angeordnet sind;
- zwei vordere Pratzen (17.2, 18.2), die oberhalb der Platte (13) vorstehen, wobei die zwei vorderen Pratzen (17.2, 18.2) auf dem vorderen Rand (14.2) und jeweils auf jeder Seite des vorderen Dorns (15.2) angeordnet sind;
wobei das hintere Flachteil (16.1) und das vordere Flachteil (16.2) derart eingerichtet sind, dass sich der prothetische Schienbeineinsatz (5) gleichzeitig gegen das hinter Flachteil (16.1) und das vordere Flachteil (16.2) stützen kann;
wobei jede hintere Pratze (17.1, 18.1) eine jeweilige Halteerweiterung (17.11, 18.11) umfasst, um den prothetischen Schienbeineinsatz (5) auf dem prothetischen Schienbeinsockel (1) zu halten, wobei jede Halteerweiterung (17.11, 18.11) ausgebildet ist, um das Einfügen unter der Halteerweiterung (17.11, 18.11) und entlang einer anterior-posterioren Richtung einer komplementären Haltelasche (57.11, 58.11), die zu dem prothetischen Schienbeineinsatz (5) gehört, zu erlauben;
wobei jede vordere Pratze (17.2, 18.2) elastische Einrastmittel des prothetischen Schienbeineinsatzes (5) auf dem prothetischen Schienbeinsockel (1) umfasst;
prothetischer Schienbeinsockel (1) **dadurch gekennzeichnet, dass** er ferner Folgendes umfasst:
- eine mediale Pratze (17.3), die oberhalb der Platte (13) vorsteht, wobei die mediale Pratze (17.3) auf dem medialen Rand (14.3) angeordnet ist, wobei die mediale Pratze (17.3) eine Halterippe (17.31) umfasst, um den prothetischen Schienbeineinsatz (5) auf dem prothetischen Schienbeinsockel (1) zu halten, wobei die Halterippe (17.31) ausgebildet ist, um das Einfügen unter der Halterippe und entlang einer medio-lateralen Richtung (Y) eines komplementären Haltezapfens, der zu dem prothetischen Schienbeineinsatz (5) gehört, zu erlauben; und
eine seitliche Pratze (17, 4), die oberhalb der Platte (13) vorsteht, wobei die seitliche Pratze (17.4) auf dem seitlichen Rand (14.4) angeordnet ist, wobei die seitliche Pratze (17.4) eine Halterippe (17.41) umfasst, um den prothetischen Schienbeineinsatz (5) auf dem prothetischen Schienbeinsockel (1) zu halten, wobei die Halterippe (17.41) ausgebildet ist, um das Einfügen unter der Halterippe (17.41) und entlang einer medio-lateralen Richtung (Y) eines komplementären Haltezapfens, der zu dem prothetischen Schienbeineinsatz (5) gehört, zu erlauben.

2. Prothetischer Schienbeinsockel (1) nach Anspruch 1, bei dem die Entfernung (D1), die das hintere Flachteil (16.1) von dem vorderen Flachteil (16.2) trennt, entlang einer antero-posterioren Richtung (X) gemessen derart bestimmt wird, dass ein Aufschrumpfen des prothetischen Schienbeineinsatzes (5) ausgeführt wird.

3. Prothetischer Schienbeinsockel (1) nach einem der vorhergehenden Ansprüche, wobei der Oberflächenzustand der Platte (13) eine mittlere arithmetische Abweichung kleiner als 0,1 µm, bevorzugt kleiner als 0,05 µm aufweist.

4. Prothetischer Schienbeinsockel (1) nach einem der vorhergehenden Ansprüche, wobei die Platte (13) eine flache Plattenoberfläche aufweist, wobei die Plattenoberfläche vorzugsweise zu dem hinteren Dorn (15.1), dem vorderen Dorn (15.2), den hinteren Pratzen (17.1, 18.1) und den vorderen Pratzen (17.2, 18.2) senkrecht ist.

5. Prothetischer Schienbeinsockel (1) nach einem der vorhergehenden Ansprüche, wobei das hintere Flachteil (16.1) und das vordere Flachteil (16.2) eben und zueinander parallel sind, wobei das hintere Flachteil (16.1) und das vordere Flachteil (16.2) vorzugsweise zu einer antero-posterioren Richtung (X) senkrecht sind.

6. Prothetischer Schienbeinsockel (1) nach Anspruch 5, wobei das hintere Flachteil (16.1) und das vordere Flachteil (16.2) derart eingerichtet sind, dass der prothetische Schienbeineinsatz (5) unter knappem Einpassen zwischen dem hinteren Flachteil (16.1) und dem vorderen Flachteil (16.2) eingefügt werden kann.

7. Prothetischer Schienbeinsockel (1) nach einem der vorhergehenden Ansprüche, wobei jede Halteerweiterung (17.11, 18.11) einen Querschnitt in Schwalbenschwanzform hat.

8. Prothetischer Schienbeinsockel (1) nach einem der vorhergehenden Ansprüche, wobei die Platte (12) aus einem Werkstoff zusammengesetzt ist, der aus der Gruppe ausgewählt ist, die aus einer Chrom-Kobaltlegierung, einem rostfreien Stahl des Typs 316L oder des Typs M30NW und einer Titanlegierung TA6V4Eli besteht.

9. Prothetischer Schienbeinsockel (1) nach einem der vorhergehenden Ansprüche, wobei eine Schutzfolie mindestens die Platte (13) überzieht, wobei die Schutzfolie derart abnehmbar ist, dass sie bei dem chirurgischen Eingriff vor dem endgültigen Eintreiben des prothetischen Schienbeineinsatzes (5) auf dem prothetischen Schienbeinsockel (1) entfernt wird.

10. Prothetischer Schienbeineinsatz (5), der dazu bestimmt ist, auf einem prothetischen Schienbeinsockel (1) stillgestellt zu sein, wobei der prothetische Schienbeineinsatz (5) Oberflächen aufweist, die ausgebildet sind, um sich mit jeweiligen Femurkondylen anzulenken, wobei der prothetische Schienbeineinsatz (5) einen unteren Abschnitt (51) hat, der Folgendes umfasst:
- eine untere Fläche (53), die ausgebildet ist, um auf einer Platte (13), die zu dem prothetischen Schienbeinsockel (1) komplementär ist, angelegt zu werden, wobei die untere Fläche (53) durch eine hintere Seite (54.1), durch eine vordere Seite (54.2), die der hinteren Seite (54.1) entgegengesetzt ist, durch eine mediale Seite (54.3) und durch eine seitliche Seite (54.4), die der medialen Seite (54.3) entgegengesetzt ist, abgegrenzt ist;
- ein hinteres Flachteil (56.1), das auf der hinteren Seite (54.1) eingerichtet ist, wobei das hintere Flachteil (56.1) entlang einer medio-lateralen Richtung (Y) auf einem medianen Bereich der hinteren Seite (54.1) angeordnet ist, wobei das hintere Flachteil (56.1) zu der vorderen Seite (54.2) bevorzugt parallel zu der Frontalebene (YZ) ausgerichtet ist;
- ein vorderes Flachteil (56.2), das auf der vorderen Seite (54.2) eingerichtet ist, wobei das vordere Flachteil (54.2) entlang einer medio-lateralen Richtung (Y) auf einem medianen Bereich der vorderen Seite (54.2) angeordnet ist, wobei das vordere Flachteil (56.2) zu der hinteren Seite (54.1) bevorzugt parallel zu der Frontalebene (YZ) ausgerichtet ist;
- zwei hintere Aufnahmen (57.1, 58.1), die auf der hinteren Seite (54.1) eingerichtet sind, die zwei hinteren Aufnahmen (57.1, 58.1) auf der einen und anderen Seite des hinteren Flachteils (56.1);
- zwei vordere Organe (57.2, 58.2), die auf der vorderen Seite (54.2) eingerichtet sind, wobei die zwei vorderen Organe (57.2, 58.2) auf der vorderen Seite (54.2) und jeweils auf der einen und anderen Seite des vorderen Flachteils (56.2) angeordnet sind;
wobei das hintere Flachteil (56.1) und das vordere Flachteil (56.2) derart eingerichtet sind, dass sich der prothetische Schienbeinsockel (1) gleichzeitig gegen das hintere Flachteil (56.1) und gegen das vordere Flachteil (56.2) anlegen kann;
wobei jede hintere Aufnahme (57.1, 58.1) eine Haltelasche (57.11, 58.11) umfasst, um den prothetischen Schienbeineinsatz (5) auf dem prothetischen Schienbeinsockel (1) zu halten, wobei jede Haltelasche (57.11, 58.11) ausgebildet ist, um entlang einer antero-posterioren Richtung (X) unter einer komplementären Halteerweiterung (17.11, 18.11), die zu dem prothetischen Schienbeinsockel (1) gehört, eingefügt zu werden;
wobei jedes vordere Organ (57.2, 58.2) elastische Einrastmittel des prothetischen Schienbeinsockels (1) auf dem prothetischen Schienbeineinsatz (5) umfasst;
prothetischer Schienbeineinsatz (5) **dadurch gekennzeichnet, dass** er außerdem Folgendes umfasst:
- einen medialen Haltezapfen, der ausgelegt ist, um sich entlang einer medio-lateralen (Y) Richtung unter eine komplementäre Halterippe (17.31), die zu einer medialen Pratze (17.3) eines prothetischen Schienbeinsockels (1) gehört, einzufügen, und
- einen seitlichen Haltezapfen, der ausgelegt ist, um sich entlang einer medio-lateralen Richtung (Y) unter eine komplementäre Halterippe (17.31), die zu einer seitlichen Pratze (17.4) eines prothetischen Schienbeinsockels (1) gehört, einzufügen.

11. Prothetischer Schienbeineinsatz (5) nach Anspruch 10, wobei mindestens der untere Abschnitt (51) aus einem Werkstoff besteht, der aus der Gruppe ausgewählt ist, die aus einem Polyethylen mit ultrahohem Molekulargewicht (UHMW-PE), Polyurethan besteht, und wobei der untere Abschnitt (51) eine Stärke (E51) größer als 1, 5 mm hat.

## Claims

1. A prosthetic tibial base (1), intended to immobilize a prosthetic tibial insert (5) configured to be articulated with femoral condyles, the prosthetic tibial base (1) comprising i) osseous attachment means (11) for attaching the prosthetic tibial base (1) onto a resected part of the tibia of a patient, and ii) a platen (12) comprising at least:
- a plate (13) configured to receive an inferior complementary face (53) of the prosthetic tibial insert (5), the plate (13) being delimited by a posterior edge (14.1), by an anterior edge (14.2) opposite the posterior edge (14.1), by a medial edge (14.3) and by a lateral edge (14.4) opposite the medial edge (14.3);
- a posterior pin (15.1) protruding above the plate (13), the posterior pin (15.1) being disposed, along a medio-lateral direction (Y), on a median region of the posterior edge (14.1), the posterior pin (15.1) having a posterior flat section (16.1) oriented towards the anterior edge (14.2), preferably parallel to the frontal plane (YZ);
- an anterior pin (15.2) protruding above the plate (13), the anterior pin (15.2) being disposed, along a medio-lateral direction (Y), on a median region of the anterior edge (14.2), the anterior pin (15.2) having an anterior flat section (16.2) oriented towards the posterior edge (14.1), preferably parallel to the frontal plane (YZ);
- two posterior lugs (17.1, 18.1) protruding above the plate (13), the two posterior lugs (17.1, 18.1) being disposed on the posterior edge (14.1) and respectively on each side of the posterior pin (15.1);
- two anterior lugs (17.2, 18.2) protruding above the plate (13), the two anterior lugs (17.2, 18.2) being disposed on the anterior edge (14.2) and respectively on each side of the anterior pin (15.2);
the posterior flat section (16.1) and the anterior flat section (16.2) are arranged in such a manner that the prosthetic tibial insert (5) may bear simultaneously against the posterior flat section (16.1) and against the anterior flat section (16.2);
each posterior lug (17.1, 18.1) includes a respective retaining extension (17.11, 18.11) for retaining the prosthetic tibial insert (5) on the prosthetic tibial base (1), each retaining extension (17.11, 18.11) being configured to allow the insertion, under the retaining extension (17.11, 18.11) and along an anteroposterior direction, of a complementary retaining tab (57.11, 58.11) belonging to the prosthetic tibial insert (5);
each anterior lug (17.2, 18.2) includes means for elastic snap-fitting the prosthetic tibial insert (5) onto the prosthetic tibial base (1);
the prosthetic tibial base (1) being **characterized in that** it further comprises:
- a medial lug (17.3) protruding above the plate (13), the medial lug (17.3) being disposed on the medial edge (14.3), the medial lug (17.3) including a retaining wing (17.31) for retaining the prosthetic tibial insert (5) on the prosthetic tibial base (1), the retaining wing (17.31) being configured to allow the insertion, under the retaining wing and along a medio-lateral direction (Y) of a complementary retaining tenon belonging to the prosthetic tibial insert (5); and
- a lateral lug (17.4) protruding above the plate (13), the lateral lug (17.4) being disposed on the lateral edge (14.4), the lateral lug (17.4) including a retaining wing (17.41) for retaining the prosthetic tibial insert (5) on the prosthetic tibial base (1), the retaining wing (17.41) being configured to allow the insertion, under the retaining wing (17.41) and along a medio-lateral direction (Y) of a complementary retaining tenon belonging to the prosthetic tibial insert (5).

2. The prosthetic tibial base (1) according to claim 1, wherein the distance (D1) separating the posterior flat section (16.1) from the anterior flat section (16.2), measured along an anteroposterior direction (X), is determined in such a manner as to press-fit the prosthetic tibial insert (5).

3. The prosthetic tibial base (1) according to any of the preceding claims, wherein the surface roughness of the plate (13) has an arithmetic mean deviation lower than 0.1 µm, preferably lower than 0.05 µm.

4. The prosthetic tibial base (1) according to any of the preceding claims, wherein the plate (13) has a planar plate surface, the plate surface being preferably perpendicular to the posterior pin (15.1), to the anterior pin (15.2), to the posterior lugs (17.1, 18.1) and to the anterior lugs (17.2, 18.2).

5. The prosthetic tibial base (1) according to any of the preceding claims, wherein the posterior flat section (16.1) and the anterior flat section (16.2) are planar and parallel, the posterior flat section (16.1) and the anterior flat section (16.2) being preferably perpendicular to an anteroposterior direction (X).

6. The prosthetic tibial base (1) according to claim 5, wherein the posterior flat section (16.1) and the anterior flat section (16.2) are arranged in such a manner that the prosthetic tibial insert (5) may be tightly inserted between the posterior flat section (16.1) and the anterior flat section (16.2).

7. The prosthetic tibial base (1) according to any of the preceding claims, wherein each retaining extension (17.11, 18.11) has a transversal section in the shape of a dovetail.

8. The prosthetic tibial base (1) according to any of the preceding claims, wherein the platen (12) is composed of a material selected from among the group consisting of cobalt-chrome alloy, type 316L or type M30NW stainless steel, and titanium alloy TA6V4Eli.

9. The prosthetic tibial base (1) according to any of the preceding claims, wherein a protective film covers at least the plate (13), the protective film being removable, in such a manner as to be removed during the surgical procedure prior to definitive impaction of the prosthetic tibial insert (5) onto the prosthetic tibial base (1).

10. A prosthetic tibial insert (5), intended to be immobilized on a prosthetic tibial base (1), the prosthetic tibial insert (5) having surfaces configured to be articulated with respective femoral condyles, the prosthetic tibial insert (5) having an inferior portion (51) comprising:
- an inferior face (53) configured to bear against a complementary plate (13) of the prosthetic tibial base (1), the inferior face (53) being delimited by a posterior side (54.1), by an anterior side (54.2) opposite the posterior side (54.1), by a medial side (54.3) and by a lateral side (54.4) opposite the medial side (54.3);
- a posterior flat (56.1) arranged on the posterior side (54.1), the posterior flat (56.1) being disposed, along a medio-lateral direction (Y), on a median region of the posterior side (54.1), the posterior flat (56.1) being oriented towards the anterior side (54.2), preferably parallel to the frontal plane (YZ);
- an anterior flat (56.2) arranged on the anterior side (54.2), the anterior flat (54.2) being disposed, along a medio-lateral direction (Y), on a median region of the anterior side (54.2), the anterior flat (56.2) being oriented towards the posterior side (54.1), preferably parallel to the frontal plane (YZ);
- two posterior housings (57.1, 58.1) arranged on the posterior side (54.1), the two posterior housings (57.1, 58.1) on either side of the posterior flat (56.1);
- two anterior members (57.2, 58.2) arranged on the anterior side (54.2), the two anterior members (57.2, 58.2) being located on the anterior side (54.2) and respectively on either side of the anterior flat (56.2);
the posterior flat (56.1) and the anterior flat (56.2) are arranged in such a manner that the prosthetic tibial base (1) may simultaneously bear against the posterior flat (56.1) and against the anterior flat (56.2);
each posterior housing (57.1, 58.1) includes a retaining tab (57.11, 58.11) for retaining the prosthetic tibial insert (5) on the prosthetic tibial base (1), each retaining tab (57.11, 58.11) being configured to be inserted, along an anteroposterior direction (X), under a complementary retaining extension (17.11, 18.11) belonging to the prosthetic tibial base (1);
each anterior member (57.2, 58.2) includes means for elastic snap-fitting the prosthetic tibial base (1) onto the prosthetic tibial insert (5);
the prosthetic tibial insert (5) being **characterized in that** it further comprises:
- a medial retaining tenon configured to be inserted, along an anteroposterior direction (X), under a complementary retaining extension (17.31) belonging to a medial lug (17.3) of a prosthetic tibial base (1); and
- a lateral retaining tenon configured to be inserted, along an anteroposterior direction (X), under a complementary retaining extension (17.31) belonging to a lateral lug (17.4) of a prosthetic tibial base (1).

11. The prosthetic tibial insert (5) according to claim 10, wherein at least the inferior portion (51) is composed of a material selected from the group consisting of ultra-high-molecular-weight polyethylene (UHMW-PE), polyurethane, and wherein the inferior portion (51) has a thickness (E51) higher than 1.5 mm.
